# EUROPÄISCHE PATENTSCHRIFT

(11) **EP 2 337 532 B1**
(45) Veröffentlichungstag und Bekanntmachung des Hinweises auf die Patenterteilung: **23.05.2012**
(21) Anmeldenummer: 09778438.3
(22) Anmeldetag: 09.09.2009
(51) Int. Cl.: A61F 9/008, A61B 5/00

(54) **Vorrichtung zur Behandlung von biologischem Gewebe mit Laserstrahlung**
Device for the treatment of biological tissue using laser radiation
Dispositif pour traiter un tissu biologique avec un faisceau laser

(30) Priorität: 09.09.2008 DE 102008046394
(43) Veröffentlichungstag der Anmeldung: 29.06.2011
(73) Patentinhaber: Medizinisches Laserzentrum Lübeck GmbH, 23562 Lübeck (DE)
(72) Erfinder: BRINKMANN, Ralf, 23562 Lübeck (DE)
(74) Vertreter: Eisenführ, Speiser & Partner
(86) Internationale Anmeldenummer: PCT/EP2009/006552
(87) Internationale Veröffentlichungsnummer: WO 2010/028822

(56) Entgegenhaltungen:
- EP-A1- 1 279 385
- WO-A1-01/97692
- KANDULLA JOCHEN ET AL: "Noninvasive optoacoustic online retinal temperature determination during continuous-wave laser irradiation." Juli 2006 (2006-07), JOURNAL OF BIOMEDICAL OPTICS 2006 JUL-AUG, VOL. 11, NR. 4, PAGE(S) 41111 , XP002553666 ISSN: 1083-3668 Seiten 41111-3 - Seiten 41111-4
- SCHLOTT K ET AL: "Optoacoustic online temperature determination during retinal laser photocoagulation" THERAPEUTIC LASER APPLICATIONS AND LASER-TISSUE INTERACTIONS III, PROCEEDINGS OF THE SPIE, Bd. 6632, 2007, Seiten 66321B-1-66321B-8, XP002553667

## Beschreibung

Die Erfindung betrifft eine Vorrichtung zur Behandlung von biologischem Gewebe, insbesondere lebendem Gewebe mit Laserstrahlung.

Bekannt ist die therapeutische Laseranwendung am Augenhintergrund, insbesondere bei Netzhauterkrankungen, zur Erzielung von Photokoagulation an der Netzhaut. Als Behandlungslaser bei der Photokoagulation kommen hauptsächlich Laser zum Einsatz, deren gepulste Behandlungsstrahlung im grünen Wellenlängenbereich liegt. Diese Strahlung wird in den Fundusschichten des Auges besonders stark absorbiert. Häufig werden Festkörperlaser, beispielsweise der frequenzverdoppelte Nd: YAG mit einer Wellenlänge von 532 nm verwendet. Auch Argon-Ionen-Laser mit 514 nm kommen häufig zur Anwendung. Die dabei zum Einsatz kommenden Laserstrahlen erzeugen Spotgrößen von 50 bis 500 µm im Zielgewebe. Die Laserleistungen können bis zu mehreren hundert mW betragen, wobei Bestrahlungsdauem von 50 ms bis 500 ms zum Einsatz kommen. Bei diabetischer Retinopathie wird mit Aussparung der Makula panretinale Photokoagulation eingesetzt, bei welcher großflächig mit einigen hundert bis zu über tausend Koagulationsspots die Behandlung durchgeführt wird. Ferner wird die Photokoagulation bei Netzhautlöchern und Netzhautabhebungen verwendet. Hierbei wird in den Randgebieten der Netzhautschäden durch Narbenbildung eine Verbindung der Retina mit dem Untergrund hergestellt.

Für die Dosierung werden bislang die Behandlungsparameter nach Erfahrungswerten eingestellt. Aufgrund unterschiedlicher Pigmentierungen im Auge können die bei der Photokoagulation erzeugten Temperaturen jedoch mehr oder weniger stark schwanken.

Aus EP 1 279 385 A1 ist die Temperaturbestimmung bei der Behandlung von biologischem Gewebe insbesondere am Augenhintergrund mittels Laserstrahlung bekannt. Dabei werden während der jeweiligen Pulse der pulsförmigen Behandlungsstrahlung zusätzliche Strahlungspulse mit geringerer Pulsdauer und geringerer Energie als bei der Behandlungsstrahlung auf das Zielgewebe gerichtet. Dabei entstehende Gewebeexpansionen und Gewebekontraktionen erzeugen bipolare Druckwellen, welche detektiert werden. Aus diesen gemessenen Drucktransienten werden unter Zuhilfenahme der Grüneisen-Eichkurve und einer Eichtemperatur die entsprechenden Temperaturwerte während der Bestrahlung ermittelt.

Ferner ist es bekannt (DE 199 16 653 A1), die im Zielgewebe generierten Drucktransienten als Messgröße der optischen Eigenschaften des Zielgewebes heranzuziehen, um den weiteren Behandlungsverlauf durch Vergleich mit speziell ermittelten Kennlinien bezüglich des Verlaufs der Veränderungen der optischen Eigenschaften während der Therapie rechnergestützt und on-line vollautomatisiert zu steuern.

Bei der Photokoagulation werden jedoch bei beginnender Denaturierung des Zielgewebes die Gewebeeigenschaften verändert, wodurch auch die durch die Messstrahlungen induzierten Druckamplituden beeinflusst werden.

Aufgabe der Erfindung ist es, eine Vorrichtung der eingangsgenannten Art zu schaffen, bei welcher die Steuerung der Behandlungsstrahlung verbessert wird.

Diese Aufgabe wird erfindungsgemäß durch eine Vorrichtung mit den Merkmalen des Patenanspruches 1 gelöst. Die Unteransprüche beinhalten vorteilhafte Weiterbildungen der Erfindung.

Die Vorrichtung beinhaltet bei der Erfindung eine Einrichtung zur Erzeugung einer gepulsten, auf ein Zielgewebe zu richtenden Behandlungsstrahlung, vorzugsweise einer Laserstrahlung. Ferner ist eine Detektoreinrichtung zur Erfassung von vom Zielgewebe augehenden Druckamplituden vorgesehen. Diese Druckamplituden können von der Behandlungsstrahlung induziert sein, wobei in diesem Fall die Behandlungsstrahlung eine Frequenz von mindestens 100 Hz aufweist. Ferner ist es möglich zusätzlich eine Messlasereinrichtung zur Erzeugung einer zusätzlichen gepulsten auf das Zielgewebe gerichteten Messstrahlung mit geringerer 'Energie und geringerer Pulsdauer als bei der Behandlungsstrahlung vorzusehen. In diesem Fall ist die Detektoreinrichtung zur Erfassung der von der Messstrahlung induzierten und vom Zielgewebe ausgehenden Druckamplituden geeignet. Ferner ist eine Auswerteeinrichtung vorgesehen zum Auswerten der von der Detektoreinrichtung erfassten Druckamplituden und eine Steuereinrichtung zur Steuerung der Behandlungsstrahlung in Abhängigkeit von den ausgewerteten Druckamplituden.

Bei der Behandlung des Zielgewebes mit der Behandlungsstrahlung insbesondere Laserstrahlung, findet während eines ersten, aufheizenden Zeitintervalls Δt₁ im Zielgewebe noch keine Gewebeänderung statt. Die Dauer dieses ersten Zeitintervalls kann beispielsweise 20 - 50 ms betragen.

Während der Behandlung mit der Behandlungsstrahlung wird vorzugsweise mit zusätzlicher gepulster Laserstrahlung eingestrahlt, wie aus EP 1 279 385 A1 bekannt ist. Die sich dabei ergebenden Druckamplituden steigen mit der Zeit t während des Intervalls Δt₁ im Mittel gemäß einer Funktion f(t) an.

Die Funktion m(t) repräsentiert das grundsätzliche Krümmungsverhalten des zeitlichen Verlaufs der mittleren Druckamplituden während der Aufheizphase des Gewebes in Abwesenheit von Gewebeveränderungen. Die Funktion m(t) ist bekannt (Jochen Kandulla, Ralf Brinkmann, "Nicht-invasive Echtzeit-Temperaturbestimmung während Laserbehandlungen an der Netzhaut des Auges": Photonic 2/2007, 42-46), ihr liegt die Errorfunktion zugrunde. Sie kann über kurze Zeitintervalle mit verschiedenen einfacheren Funktionen gut approximiert werden. Die Funktion m(t) kann in einem Speicher der Auswerteeinrichtung oder einem Speicher, welcher mit der Auswerteeinrichtung verbunden ist, gespeichert werden.

Der Fitfaktor a gemäß der Gleichung f(t)=a*m(t) ist vor der Messung unbekannt und insbesondere für jeden Bestrahlungsort in der Regel anders. Der Fitfaktor a hängt ab von der Probelaserenergie und Pigmentierung des gerade bestrahlten Areals, ebenso aber auch von der Schallausbreitung im Auge, dem akustischen Impedanzsprung an der Hornhaut, der Schallwandlergeometrie und -empfindlichkeit, der Signalverstärkung etc.

An jedem Bestrahlungsort wird der gemittelte Verlauf f(t) der im Zeitintervall Δt₁ gemessenen Druckamplituden mit dem sich dabei ergebenden Fitfaktor a gemäß der Fitbedingung f(t) = a*m(t) angefittet. Damit steht eine Funktion a*m(t) auch in einem dem ersten Zeitintervall nachfolgenden Zeitbereich Δt₂ zur Verfügung.

In Δt₂ wird zu jedem Zeitpunkt t das Verhältnis von aktueller Messdatenfunktion f(t), die den gemittelten Verlauf der gemessenen Druckamplituden wiedergibt, und der Funktion a*m(t) gebildet. Aufgrund des Rauschens der Messdaten kann in vorteilhafter Weise zu jedem Zeitpunkt ein Mittelwert der aktuellen Messwerte f(t) (z.B. aus 10 Messwerten) mit der Funktion a*m(t) ins Verhältnis gesetzt werden, etwa als V(t) = f(t)/[a*m(t)].

Für eine Gewebeänderung insbesondere Gewebekoagulation ist die Auswerteeinrichtung so ausgebildet, dass sie feststellt, ob und wann in dem dem ersten Zeitintervall folgenden zweiten Zeitintervall Δt₂ eine bestimmte vorher festgelegte Abweichung V* der aktuellen Messwerte f(t) von der Funktion auftritt (z.B. 20%, d.h. z. B. V*=0.8). Das Feststellen einer derart signifikanten Abweichung während des Zeitintervalls Δt₂ markiert erfindungsgemäß, dass Gewebeveränderungen kurz zuvor eingesetzt haben. Jedes Fortsetzen der bisherigen Bestrahlung würde sicher einen noch stärkeren Gewebeschaden erzeugen.

Aus dem Zeitpunkt tᵢ, zu dem die festgelegte Abweichung V* auftritt (also V(tᵢ)=V*), ist insbesondere die Ablaufgeschwindigkeit der Gewebedenaturierung bei fortgesetzter Bestrahlung abschätzbar. Aus dem Zeitpunkt tᵢ werden die Bestrahlungsparameter der Behandlungsstrahlung für das dritte sich an das zweite Zeitintervall anschließende Zeitintervall Δt₃ festgelegt. Diese ergeben sich aus zuvor experimentell gewonnenen Daten. Die experimentell ermittelten Daten können in einem Speicher der Auswerteeinrichtung oder in einem an die Auswerteeinrichtung angeschlossenen Speicher gespeichert sein.

Die Steuerschaltung, welche zur Steuerung der Behandlungsstrahlung dient, kann zur Steuerung der Dauer und/oder der Leistung des jeweiligen Pulses der Behandlungsstrahlung ausgebildet sein. Bei gleichbleibender Strahlungsleistung bestimmt sich die Dauer von Δt₃ vom Zeitpunkt tᵢ bzw. von der Dauer von Δt₂, wobei z.B. die Dauer von Δt₃ proportional zur Dauer von Δt₂ gewählt werden kann. Je kürzer Δt₂ ist, umso kürzer ist Δt₃.

Die Auswerteeinrichtung ist vorzugsweise als rechnergestützte Auswerteeinrichtung ausgebildet, welche entsprechende Speicher für die Funktion m(t) und die für die Steuerung der Behandlungsstrahlung, insbesondere im dritten Zeitintervall erforderlichen experimentell ermittelten Daten enthält. Hier kann es sich um Daten bezüglich der noch anzuwendenden Bearbeitungszeit und/oder der anzuwendenden Leistung der Behandlungsstrahlung handeln. Durch die Erfindung wird es ermöglicht, die gemessenen Drucktransienten für die Steuerung der Behandlungsstrahlung zu verwenden. Insbesondere ist keine Kalibrierung oder Eichung auf eine Temperatur oder andere Referenzwerte nötig.

Anhand der Figuren wird die Erfindung noch näher erläutert.

Es zeigt:
- Figur 1: ein Ausführungsbeispiel der erfindungsgemäßen Vorrichtung; und
- Figur 2: eine Kurvendarstellung zur Erläuterung der Funktionsweise des in Figur 1 dargestellten Ausführungsbeispiels;

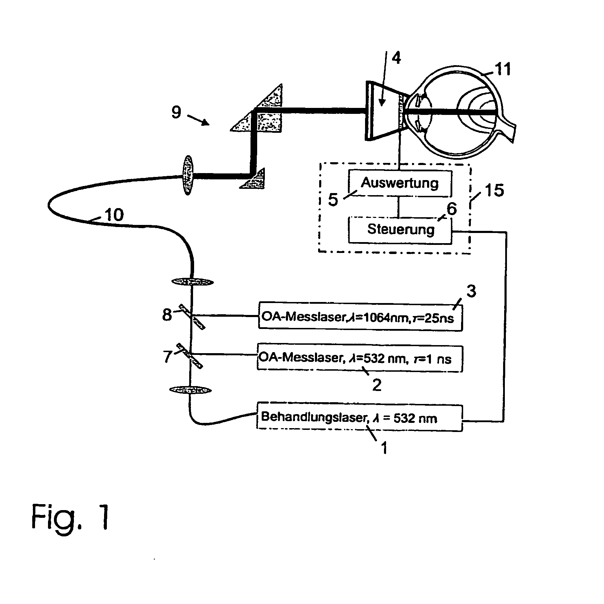

Die Figur 1 zeigt als schematisches Blockschaltbild ein Ausführungsbeispiel der Behandlungsvorrichtung. Diese beinhaltet eine Behandlungslasereinrichtung 1 welche einen Behandlungslaser aufweist, der im grünen Wellenlängenbereich, beispielsweise mit einer Wellenlänge von 532 nm eine gepulste Behandlungsstrahlung aussendet. Hierfür kann beispielsweise ein frequenzverdoppelter Nd: YAG-Laser verwendet werden. Es eignet sich auch ein Argon-Ionen-Laser, der gepulste Behandlungsstrahlung mit einer Wellenlänge von 514 nm aussendet. Die Spotgrößen für den auf das Zielgewebe auftreffenden Behandlungsstrahl können von 10 µm bis 1000 µm betragen. Die Bestrahlungszeiten für jeden Strahlungspuls können von 30 ms bis 500 ms oder auch darüber betragen. Die Laserleistung wird je nach der durchzuführenden Behandlung von 30 mW bis über 1000 mW ausgewählt werden.

Die dargestellte Vorrichtung beinhaltet ferner zwei Messlasereinrichtungen 2 und 3. Die Messlasereinrichtung 2 beinhaltet einen Messlaser, welcher im grünen Wellenlängenbereich beispielsweise mit einer Wellenlänge 532 nm eine gepulste Messlaserstrahlung aussendet. Diese Messlaserstrahlung wird mittels eines Optikkopplers 7 in den Strahlengang der Behandlungslaser-Strahlung eingekoppelt. Die zweite Messlasereinrichtung 3 beinhaltet einen Messlaser, der im Infrarotbereich beispielsweise mit einer Wellenlänge von 1064 nm gepulste Messstrahlung aussendet. Die Messstrahlung wird mittels eines Optikkopplers 8 in den Strahlengang der Behandlungsstrahlung eingekoppelt. Die Pulsenergie der beiden Messlaser ist bedeutend geringer als die der Behandlungsstrahlung und beträgt typischerweise wenige µJ. Die Pulsdauern sind ebenfalls um das mindestens hundertfache geringer als die der Behandlungsstrahlung.

Wie oben schon erläutert, werden in den aus einem Lichtleiter 10 von der Behandlungslasereinrichtung 1 kommenden Behandlungsstrahlengang mittel der Optikkoppler 7 und 8 die beiden Messstrahlungen der Messlasereinrichtungen 2 und 3 eingekoppelt. Die Behandlungsstrahlung und die Messstrahlungen werden über einen gemeinsamen Lichtleiter 10 in eine Spaltlampenoptik 9 geleitet und von dort auf den Augenhintergrund, beispielsweise die Retina eines Auges 11, gerichtet. Auf diese Weise wird erreicht, dass die Behandlungsstrahlung und die beiden Messstrahlungen im gleichen Spot auf das Zielgewebe der Retina auftreffen. Es ist jedoch auch möglich, völlig getrennte Strahlengänge und entsprechend unterschiedliche Spotdurchmesser im Auge zu verwenden.

Die von den beiden Messstrahlungen induzierten Druckwellen werden von einem Detektor 4 erfasst und gemessen. Der Detektor 4 wird mittels eines Kontaktglases auf die Augenhomhaut aufgesetzt. In EP 1 279 385 A1 sind verschiedene Detektoranordnungen beschrieben, welche verwendet werden können.

Anstelle von zwei Messlasereinrichtungen kann auch nur eine Messlasereinrichtung verwendet werden. Es ist auch möglich mehr als zwei Messlasereinrichtungen zum Einsatz zu bringen.

Es ist auch möglich ohne Messlasereinrichtungen, das heißt nur mit der Behandlungslastereinrichtung 1 zu arbeiten. Hierbei wird die Frequenz der Behandlungsstrahlung so gewählt, dass auswertbare Druckamplituden von der Detektoreinrichtung 4 erfasst werden. Die Frequenz der Behandlungsstrahlung beträgt in diesem Fall mindestens 100 Hz. Die von dieser Strahlung am Zielgewebe induzierten Druckamplituden werden dann von der Detektoreinrichtung 4 für die Auswertung erfasst.

Die von der Detektoreinrichtung 4 gemessenen Druckamplituden (Drucktransienten) werden einer Auswerteeinrichtung 5 zugeleitet und, wie noch erläutert wird, ausgewertet. In Abhängigkeit von der Auswertung erfolgt dann mittels einer Steuereinrichtung 6 die Steuerung der Behandlungslasereinrichtung, insbesondere des Behandlungslasers.

Sowohl die Auswerteeinrichtung 5 als auch die Steuereinrichtung 6 arbeiten rechnergestützt und können in einer elektronischen Rechnereinrichtung 15 implementiert sein.

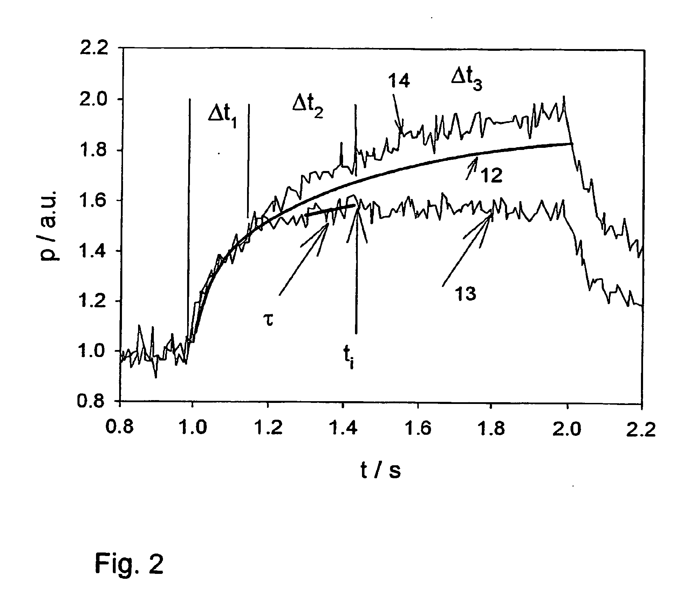

In Figur 2 sind die Verläufe der von den beiden Messstrahlungen induzierten Druckamplituden über die Zeit t in Sekunden (s) aufgetragen. Sowohl für die im grünen Wellenlängenbereich induzierten Drucktransienten als auch für die im infraroten Wellenlängenbereich induzierten Drucktransienten ergibt sich bei konstanter Pulsenergie des jeweiligen Messlasers bzw. des Behandlungslasers ein Ansteigen der Druckamplituden aufgrund der Temperaturabhängigkeit der thermoelastischen Expansion. Bei Verwendung einer Behandlungslaserleistung, welche eine Gewebekoagulation auslöst, beispielsweise einer Laserleistung von 180 mW, erfolgt innerhalb eines ersten Zeitintervalls Δt₁, in welchem noch keine Photokoagulation stattfindet, ein im wesentlichen identischer Druckanstieg bei beiden induzierten Drucktransienten 13 und 14. Die Drucktransienten 13 sind die grün induzierten Drucktransienten und die Drucktransienten 14 sind die infrarot induzierten Drucktransienten.

Bei beginnender Koagulation zeigt der Verlauf der grün induzierten Druckamplituden eine Abflachung, während die infrarot induzierten Druckamplituden weiter steigen. Der unterschiedliche Verlauf der beiden Druckkurven ergibt sich daraus, dass die infrarote Messstrahlung tiefer in die Fundusschicht eindringt als die grüne Messstrahlung. Für den grünen, stark im RPE absorbierten Strahl erhöht sich die Streuung mit einsetzender Koagulation in der Retina und dem RPE, was die Absorption im Zielgewebe reduziert. Im Infraroten tritt dieser Effekt auch auf, ist jedoch schwächer aufgrund der höheren Wellenlänge. Er wird jedoch überkompensiert durch die insgesamt erhöhte Absorption in der Choroidea durch die reduzierte mittlere freie Weglänge eines Photons. Die beginnende Photokoagulation des Gewebes führt zu Druckänderungen und Abweichungen vom erwarteten Druckverlauf ohne Phasenübergang. Dieses Verhalten wird erfindungsgemäß ausgenutzt, indem an den aktuell gemessenen Druckverlauf der Messdaten während des ersten Zeitintervalls Δt₁ eine Funktion a*m(t) mit einem möglichst einfachen Algorithmus angefittet wird. Die Kurve 12 stellt diese Funktion, welche zur Steuerung der Behandlungsstrahlung mit verwendet wird, dar. Das erste Zeitintervall Δt₁ wird so klein gewählt, dass mit Bestimmtheit keine Veränderungen des bestrahlten Gewebes eintreten. Dieses Zeitintervall kann beispielsweise 20-50 ms betragen.

Der gemittelte Verlauf f(t) der Messdaten für die von der Messlaserstrahlung im ersten Zeitintervall Δt₁ induzierten Drucktransienten wird mit einem möglichst einfachen Algorithmus, gemäß der Fitbedingung f(t) = a*m (t) angefittet. Es können natürlich auch komplexe Abbildungsalgorithmen eingesetzt werden. Der Verlauf der aktuellen MessdatenFunktion f(t)und von a*m(t) werden während der Behandlungsstrahlung an einem jeweiligen Zielgewebe ermittelt.

In dem sich an das erste Zeitintervall anschließenden zweiten Zeitintervall Δt₂ wird zu jedem Zeitpunkt das Verhältnis V aus dem mittleren Messwert f(t) für die beispielsweise grün induzierten Drucktransienten zur Funktion a*m(t), welche in der Figur 2 mit dem Bezugszeichen 12 bezeichnet ist, gebildet. Weicht das Verhältnis V zu einem Zeitpunkt tᵢ um mehr als einen vorbestimmten Verhältniswert, beispielsweise 20 % (0,2), von V=1 ab, wird das zweite Zeitintervall Δt₂ beendet.

Im dritten Zeitintervall Δt₃, welches zum Zeitpunkt tᵢ beginnt, kann auf tabellierte Daten zurückgegriffen werden, die experimentell zuvor ermittelt wurden. Es handelt sich hierbei insbesondere um Daten zur Berechnung der Dauer des dritten Zeitintervalls Δt₃ und gegebenenfalls um Daten zur Veränderung der Leistung der Behandlungsstrahlung für eine vorgewählte Koagulationsstärke.

Bei z. B. gleichbleibender Leistung der Behandlungsstrahlung wird die Dauer der Bestrahlung im dritten Zeitintervall proportional zur Dauer des zweiten Zeitintervalls Δt₂ bzw. proportional zum Zeitpunkt tᵢ gewählt.

Die in der Fitbedingung benutzte Funktion m(t) kann im Speicher des Rechners 15 abgelegt werden. Der Funktion m(t) liegt zugrunde, dass der Anstieg der Druckamplitude als Funktion der Temperatur am Bestrahlungsort in guter Nährung mit einem Polynom zweiten Grades beschreibbar ist. Die zeitliche Entwicklung der Temperaturerhöhung als Folge der Behandlungsbestrahlung ergibt sich wiederum theoretisch aus der Errorfunktion als Lösung der Wärmediffusionsgleichung. (Jochen Kandulla, Ralf Brinkmann, "Nicht-invasive Echtzeit-Temperaturbestimmung während Laserbehandlungen an der Netzhaut des Auges": Photonic 2/2007, 42-46).

### Bezugszeichenliste:

- 1: Behandlungslasereinrichtung
- 2: Messlasereinrichtung
- 3: Messlasereinrichtung
- 4: Detektoreinrichtung
- 5: Auswerteeinrichtung
- 6: Steuereinrichtung
- 7: Koppleroptik
- 8: Koppleroptik
- 9: Spaltlampengerät
- 10: Lichtleiter
- 11: Auge
- 12: Fitfunktion
- 13: Grün-induzierte Drucktransienten
- 14: Infrarot-induzierte Drucktransienten
- 15: Elektronische Rechnereinrichtung

## Patentansprüche

1. Vorrichtung zur Behandlung von biologischem, insbesondere lebendem Gewebe mit
- einer Behandlungseinrichtung (1) zur Erzeugung einer gepulsten auf ein Zielgewebe zu richtenden Behandlungsstrahlung,
- einer Detektoreinrichtung (4) zur Erfassung von vom Zielgewebe ausgehenden Druckamplituden.
- einer Auswerteeinrichtung (5) zum Auswerten der von der Detektoreinrichtung (4) erfassten Druckamplituden, und
- einer Steuereinrichtung (6) zur Steuerung der Behandlungsstrahlung in Abhängigkeit von den ausgewerteten Druckamplituden,
**dadurch gekennzeichnet, dass**
die Auswerteeinrichtung (5) ausgebildet ist,
- für eine Gewebeänderung, insbesondere Gewebekoagulation, in einem zweiten Zeitintervall Δt₂, welches einem ersten Zeitintervall Δt₁, bei dem keine Gewebeänderung am Zielgewebe stattfindet, folgt, eine Abweichung eines gemittelten Verlaufs f(t) der innerhalb des zweiten Zeitintervalls Δt₂ gemessenen Druckamplituden von einer Funktion a*m(t) zu bestimmen,
wobei m(t) ein vorab bekanntes Krümmungsverhalten des gemittelten zeitlichen Verlaufs f(t) der während des ersten Zeitintervalls Δt₁ gemessenen Druckamplituden repräsentiert und a aus der Fitbedingung f(t) = a*m(t) einer Fitprozedur am Ende von Δt₁ bestimmt wird,
- einen Zeitpunkt ti zu bestimmen, an welchem die Abweichung einen bestimmten Wert erreicht, und
- in Abhängigkeit vom bestimmten Zeitpunkt ti für ein drittes, sich an das zweite Zeitintervall Δt₂ anschließendes Zeitintervall Δt₃ Behandlungsstrahlungsparameter vorzugeben.

2. Vorrichtung nach Anspruch 1, **dadurch gekennzeichnet, dass** die Steuerschaltung (6) ausgebildet ist zur Steuerung der Dauer und/oder der Leistung des jeweiligen Pulses der Behandlungsstrahlung.

3. Vorrichtung nach einem der Ansprüche 1 bis 3, **dadurch gekennzeichnet, dass** die Auswerteeinrichtung (5) einen Speicher aufweist oder an einen zur Vorrichtung gehörenden Speicher angeschlossen ist, in welchem der Krümmungsverlauf m(t) der Funktion f(t) speichertbar

4. Vorrichtung nach einem der Ansprüche 1 bis 3, **dadurch gekennzeichnet, dass** die Auswerteeinrichtung (5) einen Speicher aufweist oder an einen zur Vorrichtung gehörenden Speicher angeschlossen ist, in welchem experimentell ermittelte Daten für die Vorgabe der Behandlungsparameter im dritten Zeitintervall Δt₃ speicherbar sind.

5. Vorrichtung nach einem der Ansprüche 1 bis 4, **dadurch gekennzeichnet, dass** bei gleichbleibender Leistung der Behandlungsstrahlung die Behandlungsdauer im dritten Zeitintervall Δt₃ proportional zur Dauer des zweiten Zeitintervalls Δt₂ ist.

6. Vorrichtung nach einem der Ansprüche 1 bis 5, **dadurch gekennzeichnet, dass** zusätzlich zur Behandlungseinrichtung (1) eine Messlasereinrichtung (2, 3) zur Erzeugung einer zusätzlichen gepulsten, auf das Zielgewebe gerichteten Messstrahlung mit geringerer Energie und geringerer Pulsdauer als bei der Behandlungsstrahlung vorgesehen ist und die Detektoreinrichtung (4) zur Erfassung der durch die Messstrahlung induzierten und vom Zielgewebe ausgehenden Druckamplituden ausgebildet ist.

7. Vorrichtung nach einem der Ansprüche 1 bis 5, **dadurch gekennzeichnet, dass** die Detektoreinrichtung (4) zur Erfassung der von der Behandlungsstrahlung induzierten und vom Zielgewebe ausgehenden Druckamplituden ausgebildet ist.

8. Vorrichtung nach Anspruch 7, **dadurch gekennzeichnet, dass** die Frequenz der Behandlungsstrahlung mindestens 100 Hz beträgt.

9. Vorrichtung nach einem der Ansprüche 1 bis 8, **gekennzeichnet durch** ihre Ausbildung zur Durchführung einer Photokoagulation am Fundus eines Auges, insbesondere am RPE.

## Claims

1. A device for the treatment of biological, in particular living, tissue, with
- a treatment means (1) for generating a pulsed treatment radiation which is to be directed onto a target tissue,
- a detector means (4) for detecting pressure amplitudes coming from the target tissue,
- an evaluation means (5) for evaluating the pressure amplitudes detected by the detector means (4), and
- a control means (6) for controlling the treatment radiation dependent on the evaluated pressure amplitudes,
**characterised in that**
the evaluation means (5) is designed
- to determine, for a tissue change, in particular tissue coagulation, in a second time interval Δt₂ which follows a first time interval Δt₁ in which no tissue change takes place on the target tissue, a deviation of an averaged gradient f(t) of the pressure amplitudes measured within the second time interval Δt₂ from a function a*m(t),
m(t) representing a previously known curvature behaviour of the averaged course over time f(t) of the pressure amplitudes measured during the first time interval Δt₁, and a being determined from the fit condition f(t) = a*m(t) of a fit procedure at the end of Δt₁,
- to determine a moment in time ti at which the deviation reaches a certain value, and
- to specify, dependent on the determined moment in time ti, treatment radiation parameters for a third time interval Δt₃ succeeding the second time interval Δt₂.

2. A device according to Claim 1, **characterised in that** the control circuit (6) is designed for controlling the duration and/or the power of the respective pulse of the treatment radiation.

3. A device according to one of Claims 1 to 3, **characterised in that** the evaluation means (5) has a memory or is connected to a memory belonging to the device in which the curvature gradient m(t) of the function f(t) can be stored.

4. A device according to one of Claims 1 to 3, **characterised in that** the evaluation means (5) has a memory or is connected to a memory belonging to the device in which experimentally ascertained data for specifying the treatment parameters in the third time interval Δt₃ can be stored.

5. A device according to one of Claims 1 to 4, **characterised in that** while the power of the treatment radiation remains the same the treatment duration in the third time interval Δt₃ is proportional to the duration of the second time interval Δt₂.

6. A device according to one of Claims 1 to 5, **characterised in that** in addition to the treatment means (1) a measurement laser means (2, 3) for generating an additional pulsed measurement radiation directed at the target tissue, of lower energy and shorter pulse duration than in the case of the treatment radiation is provided, and the detector means (4) is designed for detecting the pressure amplitudes induced by the measurement radiation and coming from the target tissue.

7. A device according to one of Claims 1 to 5, **characterised in that** the detector means (4) is designed for detecting the pressure amplitudes induced by the treatment radiation and coming from the target tissue.

8. A device according to Claim 7, **characterised in that** the frequency of the treatment radiation is at least 100 Hz.

9. A device according to one of Claims 1 to 8, **characterised by** it being designed for performing photocoagulation on the fundus of an eye, in particular on the RPE.

## Revendications

1. Installation de traitement de tissu biologique, notamment de tissu vivant, comprenant
- un dispositif (1) de traitement, pour la production d'un rayonnement de traitement pulsé à diriger sur un tissu cible,
- un dispositif (4) formant détecteur, pour la détection d'amplitudes de pression provenant du tissu cible,
- un dispositif (5) d'évaluation, pour évaluer les amplitudes de pression détectées par le dispositif (4) formant détecteur, et
- un dispositif (6) de commande, pour la commande du rayonnement de traitement en fonction des amplitudes de pression évaluées,
**caractérisée en ce que**
le dispositif (5) d'évaluation est tel
- qu'il détermine, pour une modification du tissu, notamment pour une coagulation du tissu, dans un deuxième intervalle Δt₂ de temps, qui suit un premier intervalle Δt₁ de temps, dans lequel une modification du tissu cible n'a pas eu lieu, un écart d'une courbe f(t), en moyenne, des amplitudes de pression mesurées dans le deuxième intervalle Δt₂ de temps à une fonction a*m(t),
dans lequel m(t) représente une propriété de courbure connue à l'avance de la courbe f(t), en moyenne dans le temps, des amplitudes de pression mesurées pendant le premier intervalle Δt₁ de temps et a est déterminé à partir de la condition f(t) = a*m(t) d'ajustement d'une procédure d'ajustement à la fin de Δt₁,
- qu'il détermine un instant ti ou l'écart atteint une valeur déterminée, et
- qu'il prescrit, en fonction de l'instant ti déterminé, un paramètre de rayonnement de traitement pour un troisième intervalle Δt₃ de temps se raccordant au deuxième intervalle Δt₂ de temps.

2. Installation suivant la revendication 1, **caractérisée en ce que** le circuit (6) de commande est constitué pour la commande de la durée et/ou de la puissance de l'impulsion respective du rayonnement de traitement.

3. Installation suivant l'une des revendications 1 à 2, **caractérisée en ce que** le dispositif (5) d'évaluation comporte une mémoire ou est raccordé à une mémoire appartenant à l'installation, mémoire dans laquelle peut être mémorisé le tracé m(t) de courbure de la fonction f(t).

4. Installation suivant l'une des revendications 1 à 3, **caractérisée en ce que** le dispositif (5) d'évaluation comporte une mémoire ou est raccordé à une mémoire qui fait partie de l'installation, mémoire dans laquelle des données déterminées expérimentalement sont mémorisées pour la prescription des paramètres de traitement dans le troisième intervalle Δt₃ de temps.

5. Installation suivant l'une des revendications 1 à 4, **caractérisée en ce que**, pour une même puissance du rayonnement de traitement, la durée de traitement dans le troisième intervalle Δt₃ de temps est proportionnelle à la durée du deuxième intervalle Δt₂ de temps.

6. Installation suivant l'une des revendications 1 à 5, **caractérisée en ce que**, supplémentairement au dispositif (1) de traitement, il est prévu un dispositif (2, 3) laser de mesure, pour la production d'un rayonnement de mesure supplémentaire, pulsée, dirigée sur le tissu cible, d'une énergie plus petite et d'une durée d'impulsion plus petite que le rayonnement de traitement et le dispositif (4) formant détecteur est constitué pour la détection des amplitudes de pression induites par le rayonnement de mesure et provenant du tissu cible.

7. Installation suivant l'une des revendications 1 à 5, **caractérisée en ce que** le dispositif (4) formant détecteur est constitué pour la détection des amplitudes de pression induites par le rayonnement de traitement et provenant du tissu cible.

8. Installation suivant la revendication 7, **caractérisé en ce que** la fréquence du rayonnement de traitement est d'au moins 100 Hz.

9. Installation suivant l'une des revendications 1 à 8, **caractérisée par** sa constitution pour effectuer une photo coagulation du fond de l'oeil, notamment de l'épithélium pigmentaire de la rétine.
